# EUROPÄISCHE PATENTSCHRIFT

(11) **EP 3 409 233 B1**
(45) Veröffentlichungstag und Bekanntmachung des Hinweises auf die Patenterteilung: **11.03.2020**
(21) Anmeldenummer: 18157323.9
(22) Anmeldetag: 19.02.2018
(51) Int. Cl.: A61B 90/00, G01N 1/06, B26D 1/143, B26D 1/26, B26D 3/28, B26D 5/08, B26D 7/02, A61F 2/30, A61F 2/46, A61B 17/00, A61B 17/16, A61F 11/00

(54) **MEDIZINISCHE SCHNEIDEVORRICHTUNG MIT ROTIERENDEM MESSER**
MEDICAL CUTTING DEVICE WITH A ROTARY CUTTING BLADE
DISPOSITIF DE COUPE MÉDICAL POURVU DE COUTEAU ROTATIF

(30) Priorität: 29.05.2017 DE 102017111634
(43) Veröffentlichungstag der Anmeldung: 05.12.2018
(73) Patentinhaber: Heinz Kurz GmbH, 72144 Dusslingen (DE)
(72) Erfinder: GÄCKLE, Markus, 75378 Bad Liebenzell (DE); LANG, Axel, 72770 Reutlingen (DE)
(74) Vertreter: Kohler Schmid Möbus Patentanwälte

(56) Entgegenhaltungen:
- EP-B1- 0 483 567
- DE-A1-102013 105 857
- DE-U1-202009 006 583

## Beschreibung

Die Erfindung betrifft eine medizinische Schneidevorrichtung zur Herstellung dünner Gewebe- oder Knorpelscheiben mit einem Vorrichtungskörper und einem Deckel, wobei eine erste Halteeinrichtung vorgesehen ist, die einen ersten Arbeitsabschnitt mit einer auf der Oberseite des Vorrichtungskörpers angeordneten ersten Vertiefung aufweist, die von einem ersten Begrenzungssteg ganz oder teilweise umrandet ist, wobei der Vorrichtungskörper einen Verbindungsabschnitt aufweist, an welchem der erste Arbeitsabschnitt unmittelbar starr anhängt, wobei der Deckel ein Gegenstück zum Verbindungsabschnitt aufweist, an welchem ein erster Druckabschnitt unmittelbar starr anhängt, wobei der erste Druckabschnitt im Betriebszustand der Schneidevorrichtung der ersten Vertiefung des ersten Arbeitsabschnitts mit einer durch die geometrische Formgebung des Verbindungsabschnitts sowie dessen Gegenstücks definierten, im Wesentlichen gleichmäßigen ersten Distanz gegenüberliegt, so dass ein zwischen dem ersten Arbeitsabschnitt und dem ersten Druckabschnitt verlaufender Führungsschlitz für eine Schneidklinge frei bleibt.

Eine derartige Vorrichtung ist bekannt aus DE 10 2013 105 857 B4.

### Hintergrund der Erfindung

Das Herausschneiden von dünnen körpereigenem Gewebe, insbesondere Knorpelscheiben gleichmäßiger Dicken aus einem größeren, z.B. aus der Ohrmuschel, dem Tragus, dem knorpeligen Anteil der oberen Rippenknochen oder dem Nasenseptum entnommenen Knorpelstück ist für verschiedene medizinische Zwecke eine immer wieder erforderliche Tätigkeit. Beispielsweise kann es notwendig sein, spezielle Eigenschaften des Grundkörpers näher zu untersuchen, insbesondere unter einem Mikroskop. In der Hals-Nasen-Ohren-Heilkunde werden solche dünnen Knorpelscheiben aber auch bei vielen chirurgischen Eingriffen benötigt, etwa im Mittelohr-Bereich zum Abdecken einer Mittelohr-Prothese, zum Wiederaufbau der Gehörgangshinterwand oder zur plastischen Versorgung eines Trommelfell-Defekts. Auch bei vielen nasen-chirurgischen Eingriffen werden derartige dünne Knorpelscheiben eingesetzt, um funktionelle oder ästhetische Korrekturen an der Nase durchzuführen.

In der EP 0 483 567 B1 ist eine Schneidevorrichtung beschrieben, mit welcher dünne Knorpelscheiben einer -in gewissen Grenzenvorgebbaren Dicke aus deinem größeren Knorpelstück rasch, sicher und in gleichmäßiger Qualität herausgeschnitten werden können. Um unterschiedliche Dicken der erzeugten Knorpelscheiben erzielen zu können, müssen allerdings spezielle Distanzblättchen bekannter Dicke in die Schneidevorrichtung eingelegt werden. Diese Distanzblättchen müssen -ebenso wie die Schneidevorrichtung selbst- streng gereinigt und steril gehalten und vor jeder Operation eigens entsprechend behandelt werden, was einerseits zeitaufwändig und andererseits fehleranfällig ist. Bedenkt man, dass sich in einer durchschnittlichen HNO-Klinik drei bis vier Operationssäle befinden und zu Spitzenzeiten an einem Tag fünfzehn bis zwanzig Patienten operiert werden, so kann es sein, dass an einem Tag bis zu fünfzehnmal ein Knorpelschneider bereitgestellt werden muss. Dies ist für die Sterilgutversorgung eine große logistische Herausforderung. Außerdem ist die Handhabung der Distanzblättchen nicht ganz einfach. So ist es zum Beispiel wegen ihrer geringen Größe nur bedingt möglich, sie ausreichend und gut erkennbar zu beschriften, was aber die Grundvoraussetzung dafür ist, dass während der Operation genau das Distanzblättchen mit der jeweils erforderlichen Größe bereitliegt. Auch das korrekte Einlegen und Fixieren der ziemlich kleinen Distanzblättchen in die Schneidevorrichtung erfordert einige Geschicklichkeit.

Um dünne Knorpelscheiben bestimmter unterschiedlicher Dicken in gleichmäßiger Qualität auch ohne die Verwendung der bekannten Distanzblättchen herstellen zu können, wird in der US 2010/0286693 A1 vorgeschlagen, dass -wie auch bei der bekannten Schneidevorrichtung nach EP 0 483 567 B1- die im ersten Arbeitsabschnitt auf der Oberseite des Vorrichtungskörpers angeordnete erste Vertiefung durch einen auf der Oberseite des Deckels angeordneten ersten Vorsprung verschließbar ist, wobei der erste seitliche Begrenzungssteg einen von einer Stirnseite des ersten Abschnitts her geführten, mit einem vorgegebenen ersten Abstand parallel zur Grundfläche der ersten Vertiefung verlaufenden ersten Führungsschlitz zum Einschieben einer Schneidklinge aufweist. Außerdem soll bei der Vorrichtung nach US 2010/0286693 A1 mindestens eine zweite Halteeinrichtung vorgesehen sein, die einen zweiten Abschnitt mit einer auf der Oberseite des Vorrichtungskörpers angeordneten zweiten Vertiefung aufweist, welche von einem zweiten Begrenzungssteg ganz oder teilweise umrandet und durch einen auf der Oberseite des Deckels angeordneten zweiten Vorsprung verschließbar ist, wobei der zweite seitliche Begrenzungssteg einen von einer Stirnseite des zweiten Abschnitts her geführten, mit einem vorgegebenen zweiten Abstand parallel zur Grundfläche der zweiten Vertiefung verlaufenden zweiten Führungsschlitz zum Einschieben einer Schneidklinge aufweist.

Da die Abstände zwischen dem jeweiligen Führungsschlitz und der entsprechenden Grundfläche der jeweiligen Vertiefung bei unterschiedlichen Halteeinrichtungen verschieden gewählt werden können, lassen sich mit dieser bekannten Schneidevorrichtung mit den unterschiedlichen Halteeinrichtungen Knorpelscheiben bestimmter unterschiedlicher Dicken herstellen, ohne dass dafür die beim früheren Stand der Technik unerlässlichen Distanzblättchen eingesetzt werden müssen. Weiter gibt eine Handhabung dieser Schneidevorrichtung zwischen Daumen und Zeigefinger dem Operateur mehr Sicherheit beim eigentlichen Schneidevorgang, weil alle Teile aufgrund ihrer Geometrie und ihres Designs sicher und kontrolliert zueinander bewegt werden können. Nachteilig bei dieser bekannten Schneidevorrichtung ist jedoch, dass Teile sich nur mit sehr großem Aufwand herstellen lassen. Gerade spritzgusstechnisch hergestellte Produkte haben die Eigenschaft, dass bei sehr dünnen Wandungen große Probleme entstehen können. Dies hat zur Folge, dass sich die Produkte aufgrund der finanziellen Aufwendungen in der Regel nicht wirtschaftlich realisieren lassen.

Die eingangs zitierte DE 10 2013 105 857 B4 verbessert diesen Stand der Technik, indem sie eine modifizierte -für die vorliegende Erfindung gattungsbildende- Schneidevorrichtung mit den eingangs definierten Merkmalen vorschlägt. Allerdings besteht auch bei Benutzung dieser verbesserten Vorrichtung für die handhabende Person eine gewisse Verletzungsgefahr, weil die Schneidklinge, bevor sie in den Führungsschlitz eingesteckt wird, frei zugänglich ist und daher den Benutzer bei einer ungeschickten Bewegung in den Finger schneiden kann. Ein weiterer Nachteil dieser bekannten Vorrichtung (sowie übrigens ebenso des weiter oben diskutierten Standes der Technik) liegt darin, dass das Messer zum Abschneiden der gewünschten Knorpelscheibe mit sägenden beziehungsweise hackenden Bewegungen gehandhabt werden muss, was oftmals zu ungleichmäßigen Schneideergebnissen führt. Außerdem ist bei keiner der bekannten Vorrichtungen der Schneidvorgang automatisierbar, sondern muss zwingend immer manuell ausgeführt werden.

### Aufgabe der Erfindung

Der vorliegenden Erfindung liegt demgegenüber die Aufgabe zugrunde, eine gattungsgemäße medizinische Schneidevorrichtung der eingangs beschriebenen Art mit möglichst einfachen technischen Mitteln unaufwändig und kostengünstig dahingehend zu verbessern, dass die oben geschilderten Vorteile der bekannten Schneidevorrichtungen weitestgehend beibehalten, jedoch die genannten Nachteile vermieden werden. Insbesondere soll mit der Erfindung sichergestellt werden, dass der Schneidevorgang auf einfache Weise und unfallsicher durchgeführt werden kann, wobei besonders gleichmäßige Schneideergebnisse mit am Ende qualitativ hochwertigen Schneide-Produkten erzielbar sein sollen und wobei die Schneidevorrichtung die Möglichkeit zu einer automatisierten Durchführung des Schneidvorgangs bieten soll.

### Kurze Beschreibung der Erfindung

Diese relativ komplexe Aufgabe wird, ausgehend von den im Oberbegriff des Anspruchs 1 angegebenen Merkmalen, erfindungsgemäß auf ebenso überraschend einfache wie wirkungsvolle Weise dadurch gelöst, dass bei einer gattungsgemäßen medizinischen Schneidevorrichtung die Schneidklinge innerhalb des Führungsschlitzes angeordnet und starr mit einer drehbaren Welle verbunden ist, welche rotierbar um eine senkrecht zur Ebene des Führungsschlitzes verlaufende Rotationsachse gelagert ist und durch eine Ausnehmung des Deckels senkrecht zur Ebene des Führungsschlitzes herausragt.

Durch diesen Aufbau und die damit vorgegebene Funktionsweise kann sichergestellt werden, dass die scharfe Schneidklinge bei Benutzung stets allseitig innerhalb des Führungsschlitzes verborgen bleibt, so dass keinerlei Verletzungsgefahr für eine Bedienungsperson besteht. Aufgrund der Rotation der Schneidklinge werden im Gegensatz zu den oben geschilderten Vorrichtungen nach dem Stand der Technik ganz erheblich verbesserte Arbeitsergebnisse im Hinblick auf besonders gleichmäßige Schnitte des Gewebe- oder Knorpelstücks ermöglicht. Zudem eröffnet eine rotierende -im Gegensatz zu einer lediglich einschiebbaren beziehungsweise hineindrückbaren- Klinge die Möglichkeit, an die drehbare Welle eine Krafteinleitung aus einer motorischen (in der Regel mechanischen oder elektrischen) Kraftquelle anzukoppeln um damit den Schneidvorgang automatisierbar zu gestalten, was im Endeffekt schließlich zu noch besseren, feineren und gleichmäßigeren Schneideergebnissen führt.

Besondere Vorteile der erfindungsgemäßen Vorrichtung gegenüber dem Stand der Technik sind:
Einfachheit: Das in die Vertiefung eingelegte Gewebe wird während des Schnittes in einem Formschluss in der Kavität gehalten.
Präzision: Die rotierende Klinge, die durch das Rotieren der Welle eine Schnittbewegung und eine Schnitttiefenzustellung bewirkt, erlaubt einen Schnitt durch das in der Vertiefung gehaltenen Gewebes, der sich besonders durch gleichmäßige Schnittdicke und Oberflächenqualität auszeichnet.
Sicherheit: Die Klinge ist für den Anwender nicht unmittelbar zugänglich und somit ist eine Schnittverletzung unmöglich.

### Bevorzugte Ausführungsformen der Erfindung

Ganz besonders bevorzugt ist eine Klasse von Ausführungsformen der erfindungsgemäßen Schneidevorrichtung, bei denen eine Niederhalteeinrichtung vorhanden ist, mittels welcher im Betriebszustand der Schneidevorrichtung Gewebe- oder Knorpelmaterial zur Herstellung dünner Scheiben auf die Grundfläche der ersten Vertiefung gedrückt werden kann. Handelt es sich um ein instabiles Gewebe, so kann dieses durch den Niederhalter in der Vertiefung(Kavität) gehalten und somit wieder formschlüssig in Position während des Schneidvorgangs gehalten werden.

Eine fertigungstechnisch sehr einfach herstellbare Weiterbildung dieser Ausführungsformen ist dadurch gekennzeichnet, dass die Niederhalteeinrichtung eine mechanische Höhenbegrenzung aufweist. Hierdurch kann sichergestellt werden, dass einerseits die Klingenschneide nicht in den Niederhalter schneidet und somit keine Fremdstoffe abgetragen und in Transplantat kommen, und dass andererseits dadurch noch zusätzlich das Erzeugen einer definierten Schnittdicke ermöglicht wird.

Bei einfach aufgebauten Weiterbildungen der obigen Klasse von Ausführungsformen kann die Niederhalteeinrichtung im Betriebszustand der Schneidevorrichtung manuell auf das Gewebe- oder Knorpelmaterial zur Herstellung dünner Scheiben gedrückt werden. Durch das "manuelle Niederhalten" kann der Anpressdruck auf die jeweilige individuelle Gewebekonsistenz ausgeführt werden. Erfahrene Operateure/Anwender wünschen sich häufig gezielte -manuelle- Einflussmöglichkeiten abseits vom Standard.

Alternativ kann die Niederhalteeinrichtung im Betriebszustand der Schneidevorrichtung aber auch mittels Federkraft automatisch auf das Gewebe- oder Knorpelmaterial zur Herstellung dünner Scheiben gedrückt werden. Dies ist von Vorteil, da dadurch ein gewisser Standard in der Schnittqualität und Schnittdicke, die auch von Anpresskraft abhängig ist, geschaffen werden kann.

Eine weitere Klasse von besonders einfach herzustellenden Ausführungsformen der erfindungsgemäßen Schneidevorrichtung zeichnet sich dadurch aus, dass die Rotationsachse der drehbaren Welle ortsfest in der Schneidevorrichtung angeordnet ist. Vorteilhaft sind hier insbesondere Einfachheit und Präzision: Durch ein Drehen der Rotationsachse wird die Klinge rotiert (Schneidbewegung - "Salamischnitt") und gleichzeitig die Schnitttiefenzustellung vorgenommen. Das ermöglicht eine sehr gleichmäßige Schnittdicke und Oberflächenqualität.

Bei einer alternativen Klasse von mechanisch etwas aufwändigeren Ausführungsformen ist die Rotationsachse der drehbaren Welle in der Ebene des Führungsschlitzes verschiebbar angeordnet.

Hierzu bieten sich wiederum einfachere Weiterbildungen an, bei welchen die Rotationsachse der drehbaren Welle linear in der Ebene des Führungsschlitzes verschiebbar angeordnet ist.

Etwas aufwändiger, dafür aber noch erheblich variabler gestaltbar ist demgegenüber eine Klasse von Weiterbildungen, die sich dadurch auskennzeichnen, dass die Rotationsachse der drehbaren Welle auf einer gekrümmten Bahn, vorzugsweise auf einem Kreisbahnabschnitt, in der Ebene des Führungsschlitzes verschiebbar angeordnet ist. Dadurch können mehrere Kavitäten/Vertiefungen platzsparend so angeordnet werden, dass die Produktform ergonomisch gestaltet werden kann und die Kavitäten in einer bestimmten Ausrichtung angeordnet sind und somit das Gewebe in optimaler Weise der Schneide präsentiert werden.

Besonders bevorzugt sind Varianten der oben beschriebenen Weiterbildungen, welche sich dadurch auszeichnen, dass die drehbare Welle ein zumindest teilweise in den Führungsschlitz ragendes Ritzel aufweist, welches im Betriebszustand der Schneidevorrichtung in eine im Verbindungsabschnitt angeordnete Zahnstange eingreift. Vorteilhaft sind auch hier die Einfachheit in der Bedienung, die unaufwändige Herstellung sowie eine perfekte Kombination zwischen Rotation (Schneidgeschwindigkeit) und Vorschub (Schnitttiefenzustellung).

Vorteilhaft sind auch Ausführungsformen der erfindungsgemäßen Schneidevorrichtung, bei denen an dem der Schneidklinge abgewandten Ende der drehbaren Welle außerhalb des Deckels ein Drehknopf starr mit der drehbaren Welle verbunden ist. Besonders hervorzuheben sind hier die hohe Ergonomie und der lange Hebel um ein gutes Drehmoment für Schnitt und Vorschub zu gewährleisten.

Von besonders großem praktischen Vorteil sind auch Ausführungsformen der Erfindung, bei welchen an dem der Schneidklinge abgewandten Ende der drehbaren Welle außerhalb des Deckels ein Eingriffsteil zum motorischen Antrieb der drehbaren Welle starr mit dieser verbunden ist. Damit kann insbesondere der Schneidevorgang mechanisiert und sogar automatisiert werden. Damit erreicht man insbesondere eine sehr hohe Gleichmäßigkeit des Schnittes und dadurch wiederum eine erhöhte Präzision und eine hohe Oberflächengüte.

Die rotierbare Schneidklinge der erfindungsgemäßen Schneidevorrichtung kann bei zahlreichen Ausführungsformen geometrisch ganz unterschiedlich geformt sein. So kann die Schneidklinge -je nach speziellem Einsatzzweck- eine ovale oder eine sichelartige oder eine balkenartige oder eine kreisrunde Form aufweisen, insbesondere auf einem Kreisabschnitt, vorzugsweise auf einem Viertel- oder Halbkreis. Die Klinge kann einen einseitigen oder einen zweiseitigen Schliff besitzen und gerade, aber auch eine gebogene Schneide aufweisen. Je nach Gewebeart, Gewebekonsistenz und beabsichtigter Präzision kann eine optimal geeignete Klingenform verwendet werden.

Ganz besonders bevorzugt ist Klasse von Ausführungsformen der erfindungsgemäßen Schneidevorrichtung, bei denen die Halteeinrichtung nicht nur eine einzige, sondern eine Vielzahl von Vertiefungen aufweist, die im Betriebszustand der Schneidevorrichtung unterhalb des Führungsschlitzes angeordnet sind. Somit werden mit einer einzigen Vorrichtung mehrere verschiedene Schnittdicken, also Gewebescheibendicken, ermöglicht, ohne dass die Vorrichtung oder ein Teil der Vorrichtung gewechselt werden muss.

Bei Weiterbildungen dieser Ausführungsformen können die Vertiefungen geometrisch ganz unterschiedlich gestaltet sein, insbesondere hinsichtlich ihrer jeweiligen Tiefe und/oder hinsichtlich ihrer jeweiligen Bodenfläche bezüglich Form und/oder Flächengröße und/oder konkaver, ebener oder konvexer Ausgestaltung und/oder hinsichtlich ihres jeweiligen Begrenzungsstegs bezüglich Form und/oder Größe. Damit können dann völlig unterschiedlich geformte Schneidprodukte in verschiedenen Größenklassen hergestellt werden. Gewebescheiben mit definierter 3D-Form können so einfach und schnell erzeugt werden. Dies war bisher so nicht möglich.

Bei etwas komplexeren Ausführungsformen der erfindungsgemäßen Schneidevorrichtung, die sich besonders zu einer zeitgleichen Verarbeitung einer Vielzahl von Schneideprodukten eignen, hängt an dem Verbindungsabschnitt gegenüber dem ersten Arbeitsabschnitt mindestens ein zweiter Arbeitsabschnitt, insbesondere auch ein dritter Arbeitsabschnitt, jeweils mit dem gleichen gemeinsamen Führungsschlitz an, wobei jeder Arbeitsabschnitt mindestens eine Vertiefung aufweist, und wobei die Vertiefungen auf einer Linie oder auf einer gekrümmten Kurve in einer parallel zur Ebene des Führungsschlitzes verlaufenden Ebene angeordnet sind.

Je nach speziellem Einsatzzweck können die Komponenten der erfindungsgemäßen Schneidevorrichtung in ganz unterschiedlichen geometrischen Ausgestaltungen ausgebildet sein. So kann etwa der Begrenzungssteg einen kreisförmigen oder einen ovalen oder einen polygonalen, insbesondere quadratischen, Querschnitt in einer parallel zur Ebene des Führungsschlitzes verlaufenden Ebene aufweisen. Insbesondere können damit bestimmte vorpräparierte Gewebeschabschnitte besser formstabil gehalten werden.

Bei einer Klasse von Ausführungsformen der erfindungsgemäßen Schneidevorrichtung sind der Vorrichtungskörper und der Deckel im Betriebszustand der Schneidevorrichtung lösbar miteinander verbunden. So könnte auch ein wiederverwendbares Gerät hergestellt werden, welches sich im zerlegten Zustand reinigen und desinfizieren lässt.

Alternativ können aber bei einer dazu alternativen Klasse von -besonders einfach handhabbaren - Ausführungsformen sind der Vorrichtungskörper und der Deckel im Betriebszustand der Schneidevorrichtung unlösbar miteinander verbunden, insbesondere verschweißt, verklebt oder dauerhaft verklemmt. Von Vorteil ist hier besonders die Sicherheit für den Anwender, da keine Montage von scharfen Klingen vom Anwender vorgenommen werden muss und somit keine Schnittverletzung besteht. Eine erhöhte Sicherheit für den Patienten ergibt sich hier durch ein steriles Einweggerät. Damit besteht keine Gefahr von Kontaminationsverschleppung etwa durch ungenügendes Reinigen und Sterilisieren durch den Anwender.

Hinsichtlich der Materialauswahl sind bei der erfindungsgemäßen Schneidevorrichtung kaum Grenzen gesetzt. Bei bevorzugten Ausführungsformen sind der Vorrichtungskörper und der Deckel aus Kunststoff oder Metall und die rotierende Schneidklinge aus Metall, Keramik oder Metallkeramik gefertigt.

Gegenüberliegende Flächen der Druckplatten können aufgeraut, geriffelt oder genoppt sein, um ein Abrutschen des zu bearbeitenden Gewebestückes beziehungsweise Knorpels zu verhindern.

Eine weitere bevorzugte Klasse von Ausführungsformen der erfindungsgemäßen Schneidevorrichtung zeichnet sich dadurch aus, dass an den Arbeitsabschnitten des Vorrichtungskörpers und/oder an den entsprechenden Abschnitten des Deckels, welche die Vorsprünge tragen, Kennzeichnungen angebracht sind, die den durch die jeweilige Distanz vorgegebenen Abstand des Führungsschlitzes zur Grundfläche der entsprechenden Vertiefung und somit die mit der jeweiligen Halteeinrichtung erzielbare Dicke der herzustellenden Knorpelscheibe angeben.

Bei Weiterbildungen dieser Ausführungsformen können die Kennzeichnungen Zahlen umfassen, die den jeweiligen vorgegebenen Abstand des Führungsschlitzes zur Grundfläche der entsprechenden Vertiefung und somit die mit der jeweiligen Halteeinrichtung erzielbare Dicke der herzustellenden Knorpelscheibe im metrischen Maßsystem, insbesondere in Millimetern, oder im zöllischen Maßsystem, insbesondere in Inch, angeben.

Alternativ oder ergänzend können die Kennzeichnungen aber auch graphische Darstellungen, insbesondere Skalenstriche, Punkte und dergleichen umfassen, die den jeweiligen vorgegebenen Abstand des Führungsschlitzes zur Grundfläche der entsprechenden Vertiefung und somit die mit der jeweiligen Halteeinrichtung erzielbare Dicke der herzustellenden Knorpelscheibe angeben.

In der Handhabung besonders günstig sind Varianten der oben beschriebenen Ausführungsformen, bei denen die Kennzeichnungen auf der die Vertiefungen tragenden Oberseite des Vorrichtungskörpers und/oder auf der der (oder den) Druckplatte(n) gegenüberliegenden Unterseite des Deckels angebracht sind.

Bei einer weiteren ergonomisch günstigen Ausführungsform der erfindungsgemäßen Schneidevorrichtung sind auf der den Vertiefungen gegenüberliegenden Unterseite des Vorrichtungskörpers und/oder auf der den Druckplatten gegenüberliegenden Unterseite des Deckels konvexe und/oder konkave Griffhilfen angebracht, die eine Orientierungshilfe zur Ausübung eines Druckes auf das jeweilige Zentrum der Vorsprünge bieten.

Auf der den Druckplatten gegenüberliegenden Unterseite des Deckels kann bei Ausführungsformen der Erfindung eine randseitig umlaufende Umrandung vorgesehen sein.

Ebenso kann bei weiteren Ausführungsformen auf der Unterseite des Vorrichtungskörpers eine umlaufende Wandung vorgesehen sein, die einen oder mehrere Arbeitsräume umschließt, welche beispielsweise zur Vorbearbeitung eines Knorpelstücks vor dem Abschneiden der gewünschten Scheibe oder zur weiteren Bearbeitung der abgeschnittenen Knorpelscheibe dienen können.

Der Bearbeitung von abgeschnittenen Knorpelscheiben können auch runde und/oder ovale Templates unterschiedlichen Durchmessers dienen, welche bei Ausführungsformen der erfindungsgemäßen Schneidevorrichtung in einer Fläche der Schneidevorrichtung, insbesondere in einem der Arbeitsräume auf der den Vertiefungen gegenüberliegenden Unterseite des Vorrichtungskörpers und/oder auf der den Druckplatten gegenüberliegenden Unterseite des Deckels eingearbeitet sind.

Ganz besonders vorteilhaft sind Ausführungsformen der erfindungsgemäßen Schneidevorrichtung, bei denen in einer Fläche der Schneidevorrichtung, insbesondere auf der den Vertiefungen gegenüber liegenden Unterseite des Vorrichtungskörpers, vorzugsweise in einem Arbeitsraum, und/oder auf der den Druckplatten gegenüber liegenden Unterseite des Deckels eine Mess-Skala eingearbeitet ist, mit deren Hilfe die zu verarbeitenden Knorpelstücke und/oder die abgeschnittenen Knorpelscheiben problemlos vermessen werden können. Auch können hier runde oder ovale Templates unterschiedlichen Durchmessers zur Feinbearbeitung der Knorpelstücke eingearbeitet sein.

Die erfindungsgemäße Schneidevorrichtung kann insbesondere auch als preisgünstiges Einweg-Produkt ausgeführt werden, welches nach einmaliger Benutzung entsorgt wird. Damit eine Reinigung und erneute Sterilisation der Vorrichtung. Dies wird mehr und mehr wichtig, da die Verkeimung von chirurgischen Instrumenten ein immer größer werdendes Problem innerhalb des Klinikalltags darstellt. Daher ist die erfindungsgemäße Schneidevorrichtung ganz besonders bevorzugt aus einem sterilisierbaren Kunststoff hergestellt. Damit lässt sich die Schneidevorrichtung ganz erheblich preisgünstiger herstellen als die üblichen Vorrichtungen aus Metall. Die Anlieferung zur Operation erfolgt dann in einer Sterilverpackung und die benutzte Schneidevorrichtung kann einfach entsorgt werden. Ein solches steril verpacktes Einmal-Produkt hat außerdem den Vorteil, dass nicht vor jeder Operation eine aufwändige Reinigung und Sterilisation der Schneidevorrichtung erfolgen muss und es minimiert sich auch das Risiko einer Infektion, dass bei einer Sterilgutversorgung nicht ausgeschlossen werden kann. Vorzugsweise wird die Schneidevorrichtung in einem Spritzgussverfahren hergestellt.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen.

Die einzelnen Merkmale können je einzeln für sich oder zu mehreren in beliebigen Kombinationen bei Varianten der Erfindung verwirklicht sein. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

In der schematischen Zeichnung sind Ausführungsbeispiele der Erfindung dargestellt, welche in der nachfolgenden Beschreibung näher erläutert werden.

Im Einzelnen zeigen:
- Fig. 1a: eine schematische räumliche Darstellung schräg auf die Oberseite des Vorrichtungskörpers einer Ausführungsform der erfindungsgemäßen Schneidevorrichtung mit zwei Vertiefungen, mit einer diese verbindenden Zahnstange sowie mit einem gerändelten Drehknopf am oberen Ende der drehbaren Welle;
- Fig. 1b: wie Fig. 1a, jedoch mit rotierender, kreisrunder Schneidklinge auf der Welle;
- Fig. 1c: die Ausführungsform von Fig. 1b mit auf den Vorrichtungskörper aufgesetzten Deckel sowie mit Niederhalteeinrichtung in der ersten Vertiefung;
- Fig. 2a: eine schematische Schrägansicht einer sehr einfachen Ausführungsform mit balkenförmiger, asymmetrisch rotierender Schneidklinge und einer einzigen Vertiefung;
- Fig. 2b: die Ausführungsform von Fig. 2a, jedoch ohne Deckel;
- Fig. 3a: eine Schrägansicht auf eine Ausführungsform mit kreisförmiger Schneidklinge sowie drei linear angeordneten Vertiefungen auf dem Vorrichtungskörper bei aufgesetztem Deckel;
- Fig. 3b: die Ausführungsform von Fig. 3a mit teilweise abgenommenem Deckel;
- Fig. 3c: die Ausführungsform von Fig. 3b in Draufsicht von oben;
- Fig. 3d: eine Ausführungsform mit drei auf einer gekrümmten Kurve angeordneten Vertiefungen auf dem Vorrichtungskörper in einer Draufsicht wie Fig. 3c;
- Fig. 4a: eine Schrägansicht auf eine Ausführungsform mit drei linear angeordneten Vertiefungen auf dem Vorrichtungskörper bei aufgesetztem Deckel und mit einer Ausnehmung als Eingriffsteil zum motorischen Antrieb am oberen Ende der drehbaren Welle;
- Fig. 4b: wie >Fig. 4a, aber mit einem Ritzel als Eingriffsteil für einen motorischen Antrieb am oberen Ende der drehbaren Welle; und
- Fig. 5: eine schematische Ansicht diverser Messerformen.

Die in den Figuren der Zeichnung schematisch räumlich dargestellten Ausführungsformen der erfindungsgemäßen medizinischen **Schneidevorrichtung 10; 20; 30; 40** dienen zur Herstellung dünner Gewebe- oder Knorpelscheiben aus einem größeren Knorpelstück und sind aus einem sterilisierbaren Material, vorzugsweise aus sterilisierbarem Kunststoff insbesondere in einem Spritzgussverfahren, möglicherweise auch aus Metall hergestellt. Sie umfassen einen **Vorrichtungskörper 11; 21; 31; 41** und einen **Deckel 12; 22; 32; 42,** wobei eine erste Halteeinrichtung vorgesehen ist, die einen **ersten Arbeitsabschnitt 11a'** mit einer auf der Oberseite des Vorrichtungskörpers 11; 21 angeordneten **ersten Vertiefung 13a; 23a; 33a; 43a** aufweist, welche von einem **ersten Begrenzungssteg 14** ganz -sowie bei in der Zeichnung nicht dargestellten Ausführungsformen wahlweise auch jeweils durch mehrere Einzelstege nur teilweise- umrandet ist. Der Begrenzungssteg 14 kann einen kreisförmigen oder einen ovalen oder einen polygonalen, insbesondere quadratischen, Querschnitt in einer parallel zur Ebene des Führungsschlitzes 17 verlaufenden Ebene aufweisen.

Der Vorrichtungskörper 11; 21; 31; 41 und der Deckel 12; 22; 32; 42 sind im Betriebszustand der Schneidevorrichtung entweder lösbar miteinander verbunden oder unlösbar miteinander verschweißt, verklebt oder dauerhaft verklemmt.

Der Vorrichtungskörper 11; 21 weist einen **Verbindungsabschnitt 19'** auf, an welchem der erste Arbeitsabschnitt 11a' unmittelbar starr anhängt. Der Deckel 12; 22; 32, 42 weist ein **Gegenstück 19"** zum Verbindungsabschnitt 19' auf, an welchem ein erster **Druckabschnitt 11a"** unmittelbar starr anhängt. Der erste Druckabschnitt 11a" liegt im Betriebszustand der Schneidevorrichtung 10; 20; 30; 40 der ersten Vertiefung 13a; 23a; 33a; 43a des ersten Arbeitsabschnitts 11a' mit einer durch die geometrische Formgebung des Verbindungsabschnitts 19' sowie dessen Gegenstücks 19" definierten, im Wesentlichen gleichmäßigen ersten Distanz dₐ gegenüber, so dass ein zwischen dem ersten Arbeitsabschnitt 11a' und dem ersten Druckabschnitt **11a"** verlaufender **Führungsschlitz 17; 27; 37; 47** für eine **Schneidklinge 18; 28; 38; 48** aus Metall, Keramik oder Metallkeramik frei bleibt, wobei der durch die Distanz dₐ vorgegebene Abstand des Führungsschlitzes 17; 27; 37; 47 zur Grundfläche der ersten Vertiefung 13a; 23a; 33a; 43a die mit der ersten Halteeinrichtung erzielbare Dicke einer mittels der Schneidevorrichtung 10; 20; 30; 40 herzustellenden Gewebe- oder Knorpelscheibe definiert.

Erfindungsgemäß sind die Schneidevorrichtungen 10; 20; 30; 40 dadurch gekennzeichnet, dass die Schneidklinge 18; 28; 38; 48 innerhalb des Führungsschlitzes 17; 27; 37; 47 angeordnet und starr mit einer **drehbaren Welle 15; 25; 35; 45** verbunden ist, welche rotierbar um eine senkrecht zur Ebene des Führungsschlitzes 17; 27; 37; 47 verlaufende Rotationsachse gelagert ist und durch eine Ausnehmung des Deckels 12; 22; 32; 42 senkrecht zur Ebene des Führungsschlitzes 17; 27; 37; 47 herausragt.

In der Regel ist bei der erfindungsgemäßen Schneidevorrichtung 10; 20; 30; 40 eine **Niederhalteeinrichtung 16** vorhanden, mittels welcher im Betriebszustand der Schneidevorrichtung Gewebe- oder Knorpelmaterial zur Herstellung dünner Scheiben auf die Grundfläche der ersten Vertiefung 13a; 23a; 33a; 43a gedrückt werden kann. Die Niederhalteeinrichtung 16 kann eine -in der Zeichnung nicht näher dargestellte- mechanische Höhenbegrenzung aufweisen und wird im Betriebszustand der Schneidevorrichtung entweder manuell oder mittels Federkraft automatisch auf das Gewebe- oder Knorpelmaterial zur Herstellung dünner Scheiben gedrückt.

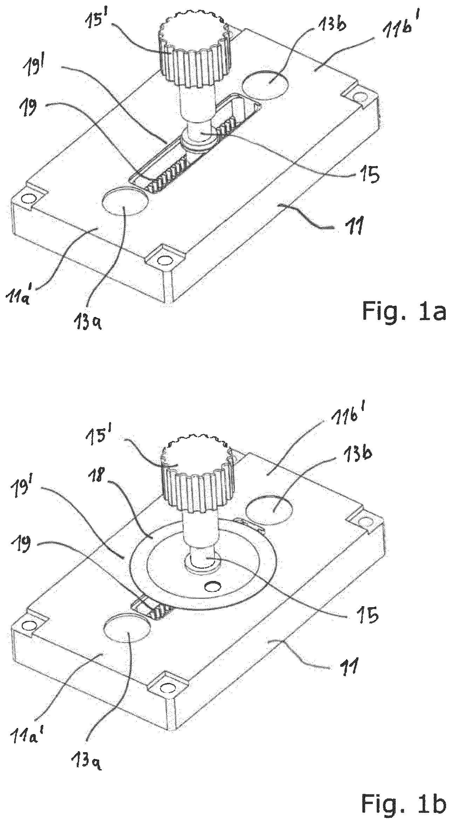

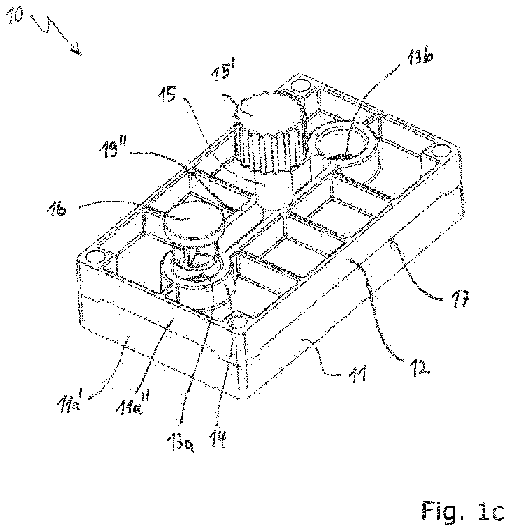

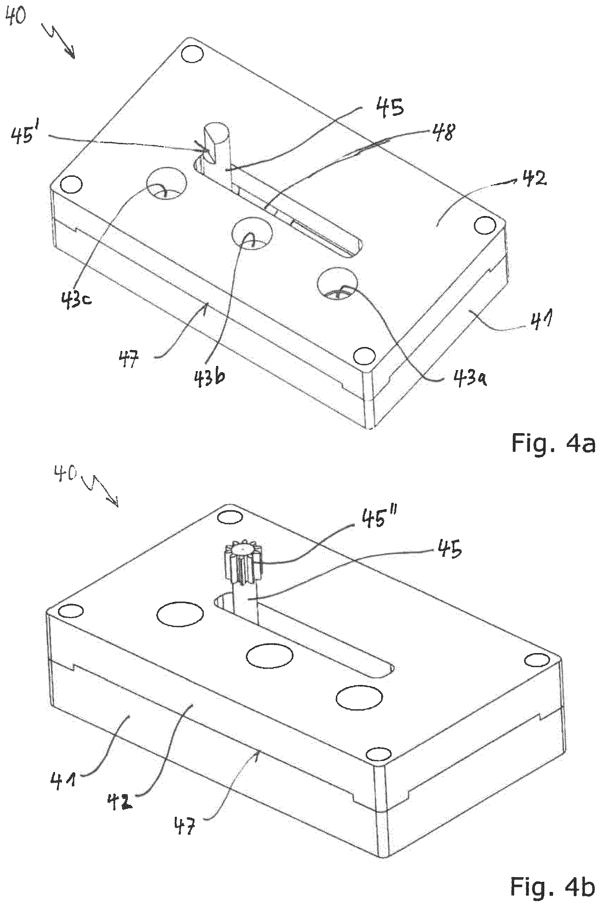

An dem der Schneidklinge 18; 28; 38 abgewandten Ende der drehbaren Welle 15; 25; 35 kann außerhalb des Deckels 12; 22; 32 ein **Drehknopf 15'; 25'; 35'** starr mit der drehbaren Welle 15; 25; 35 verbunden sein, wie in den Figuren 1a-c, 2a,b sowie 3a-d dargestellt. Die Figuren 4a,b zeigen Ausführungsformen, bei welchen an dem der Schneidklinge 48 abgewandten Ende der drehbaren Welle 45 außerhalb des Deckels 42 ein **Eingriffsteil 45'; 45"** zum motorischen Antrieb der drehbaren Welle 45 starr mit dieser verbunden ist. In Fig. 4a ist dieses Eingriffsteil 45' als Stufe am oberen Ende der Welle 45 ausgeführt. Insbesondere kann das Eingriffsteil 45" auch die Form eines Zahnrads oder Ritzels haben, wie in Fig. 4b dargestellt.

Die Figuren 1a-c, 3a-d sowie 4a,b zeigen Ausführungsformen der erfindungsgemäßen Schneidevorrichtung 10; 30; 40, bei welchen jeweils die Halteeinrichtung im Betriebszustand der Schneidevorrichtung unterhalb des Führungsschlitzes 17; 37; 47 angeordnete **weitere Vertiefungen 13b; 33b; 43b; 33c; 43c** aufweist. Die Vertiefungen können geometrisch unterschiedlich gestaltet sein, insbesondere hinsichtlich ihrer jeweiligen Tiefe und/oder hinsichtlich ihrer jeweiligen Bodenfläche bezüglich Form und/oder Flächengröße und/oder konkaver, ebener oder konvexer Ausgestaltung und/oder hinsichtlich ihres jeweiligen Begrenzungsstegs bezüglich Form und/oder Größe.

Bei derartigen Ausführungsformen mit einer Vielzahl von Vertiefungen 13a; 33a; 43a; 13b; 33b; 43b; 33c; 43c ist es vorteilhaft, wenn die Rotationsachse der drehbaren Welle 15; 35; 45 in der Ebene des Führungsschlitzes 17; 37; 47 verschiebbar angeordnet ist, um mit nur einer einzigen Schneidklinge 18; 38; 48 alle vorhandenen Vertiefungen zu erreichen.

Die Figuren 1a-c, 3a-c sowie 4a,b zeigen Ausführungsformen mit linearer Verschiebbarkeit der drehbaren Welle 15; 35; 45. In der Zeichnung nicht eigens dargestellt sind Ausführungsformen der Erfindung, bei welchen die Rotationsachse der drehbaren Welle auf einer gekrümmten Bahn, etwa auf einem Kreisbahnabschnitt, in der Ebene des Führungsschlitzes verschiebbar ist.

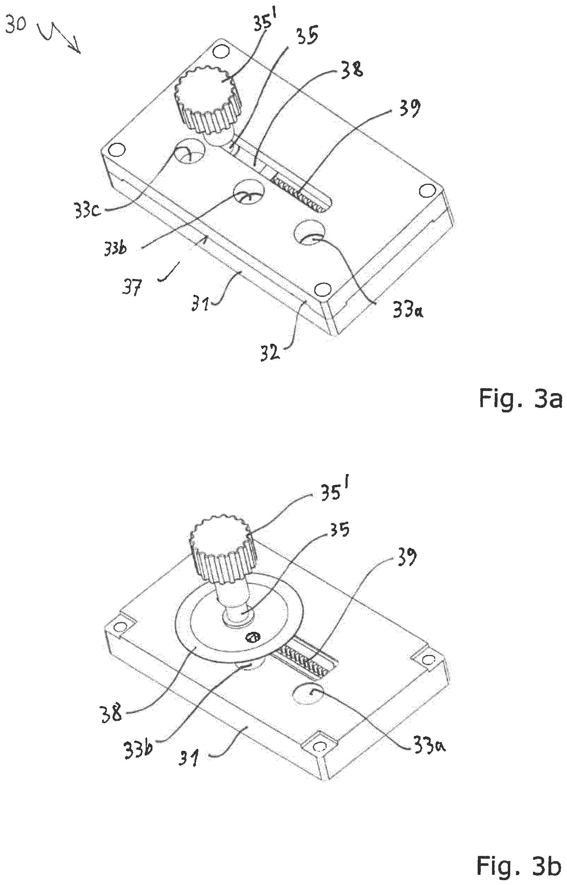

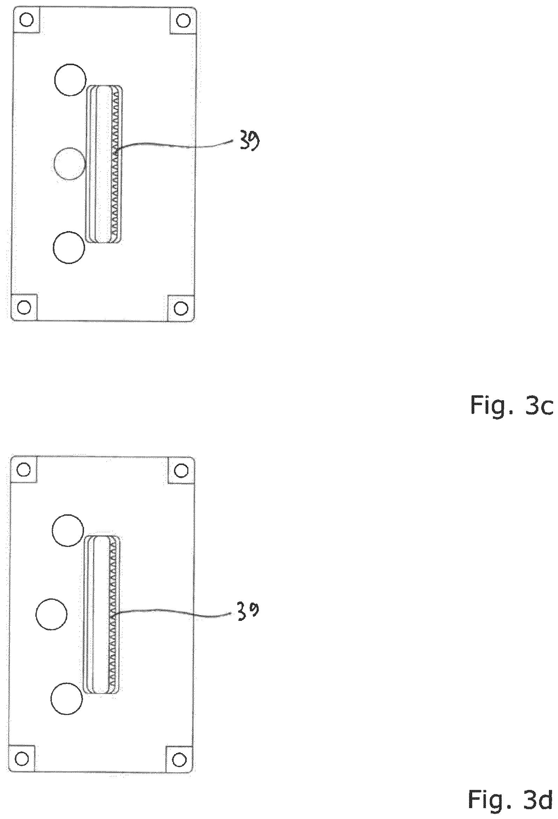

Bei den in den Fign. 1a,b sowie 3a-d gezeigten Ausführungsformen der erfindungsgemäßen Schneidevorrichtung 10; 30 erkennt man zudem jeweils eine im Verbindungsabschnitt 19' angeordnete **Zahnstange 19; 39.** In letztere kann ein zumindest teilweise in den Führungsschlitz 17; 37 ragendes, auf der drehbaren Welle 15; 35 angeordnetes Ritzel eingreifen, welches aus Gründen der Übersichtlichkeit in der Zeichnung nicht eigens dargestellt ist.

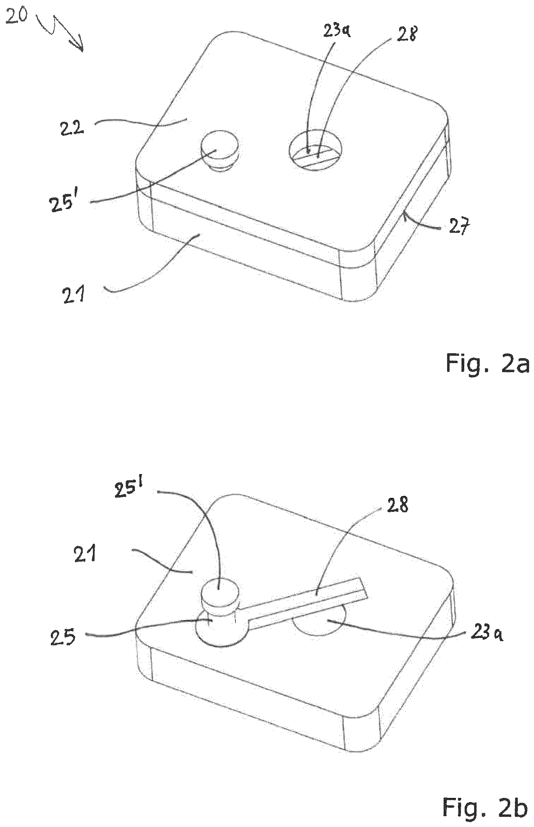

Die Rotationsachse der drehbaren Welle 25 der Ausführungsform gemäß den Fign. 2a,b ist demgegenüber ortsfest in der Schneidevorrichtung 20 angeordnet. Da bei dieser sehr einfachen Ausführungsform lediglich eine einzige Vertiefung 23a vorhanden ist, wäre eine Verschiebbarkeit der Welle 25 in der Regel auch nicht sinnvoll.

Außer der ersten Halteeinrichtung zum Halten des Gewebe- oder Knorpelstücks beim Zuschneiden einer gewünschten Scheibe können -wie bei den Ausführungsformen gemäß den Fign. 1a-c, 3a-d sowie 4a,b gezeigt- auch eine zweite Halteeinrichtung und bei in der Zeichnung nicht dargestellten Ausführungsformen der Erfindung sogar noch eine dritte sowie gegebenenfalls auch noch weitere Halteeinrichtungen vorgesehen sein. Bei drei oder vier Halteeinrichtungen werden diese aus ergonomischen Gründen relativ zueinander in Kreuzform angeordnet sein.

An dem Verbindungsabschnitt 19' gegenüber dem ersten Arbeitsabschnitt 11a' ist etwa bei der Ausführungsform nach den Fign. 1a-c ein **zweiter Arbeitsabschnitt 11b'** -oder wie bei den Ausführungsformen der Fign. 3a-d sowie 4a,b auch noch ein dritter Arbeitsabschnitt- jeweils mit dem gleichen gemeinsamen Führungsschlitz 17; 37; 47 anhängt, wobei jeder Arbeitsabschnitt 11a'; 11b' mindestens eine Vertiefung 13a, 13b; 33a, 33b, 33c; 43a, 43b, 43c aufweist, und wobei die Vertiefungen 13a; 13b auf einer Linie oder auf einer gekrümmten Kurve in einer parallel zur Ebene des Führungsschlitzes 17; 37; 47 verlaufenden Ebene angeordnet sind.

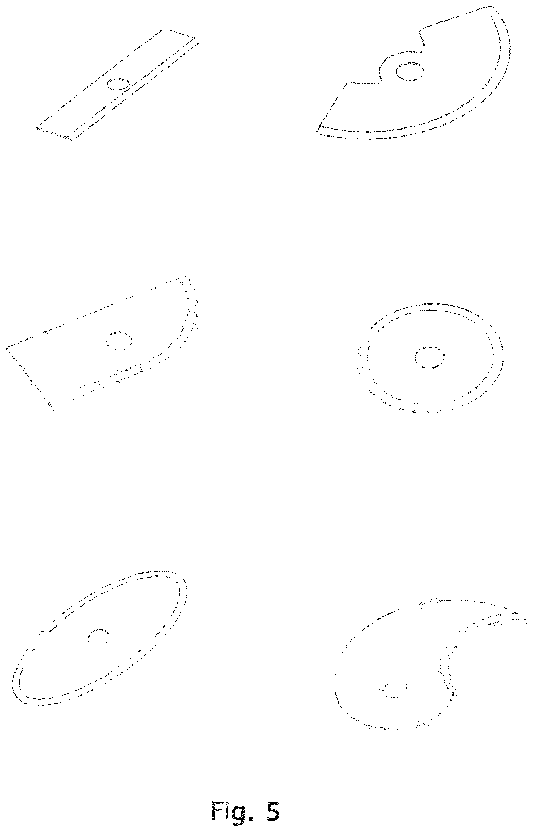

Die rotierbare Schneidklinge 18; 28; 38; 48 der erfindungsgemäßen Schneidevorrichtung 10; 30; 40 kann unzählige verschiedene Formen je nach speziellem Einsatzzweck aufweisen, etwa eine ovale oder eine sichelartige oder eine balkenartige oder eine kreisrunde Form, insbesondere auf einem Kreisabschnitt, vorzugsweise auf einem Viertel- oder Halbkreis. Eine kleine Auswahl derartiger möglicher Formen der rotierbaren Schneidklinge ist in Fig. 5 dargestellt.

## Patentansprüche

1. Medizinische Schneidevorrichtung (10; 20; 30; 40) zur Herstellung dünner Gewebe- oder Knorpelscheiben mit einem Vorrichtungskörper (11; 21; 31; 41) und einem Deckel (12; 22; 32; 42), wobei eine erste Halteeinrichtung vorgesehen ist, die einen ersten Arbeitsabschnitt (11a') mit einer auf der Oberseite des Vorrichtungskörpers (11; 21; 31; 41) angeordneten ersten Vertiefung (13a; 23a; 33a; 43a) aufweist, die von einem ersten Begrenzungssteg (14) ganz oder teilweise umrandet ist, wobei der Vorrichtungskörper (11; 21; 31; 41) einen Verbindungsabschnitt (19') aufweist, an welchem der erste Arbeitsabschnitt (11a') unmittelbar starr anhängt, wobei der Deckel (12; 22; 32; 42) ein Gegenstück (19") zum Verbindungsabschnitt (19') aufweist, an welchem ein erster Druckabschnitt (11a") unmittelbar starr anhängt, wobei der erste Druckabschnitt (11a") im Betriebszustand der Schneidevorrichtung der ersten Vertiefung (13a; 23a; 33a; 43a) des ersten Arbeitsabschnitts (11a') mit einer durch die geometrische Formgebung des Verbindungsabschnitts (19') sowie dessen Gegenstücks (19") definierten, im Wesentlichen gleichmäßigen ersten Distanz gegenüberliegt, so dass ein zwischen dem ersten Arbeitsabschnitt (11a'; 21') und dem ersten Druckabschnitt (11a") verlaufender Führungsschlitz (17; 27; 37; 47) für eine Schneidklinge (18; 28; 38; 48) frei bleibt,
**dadurch gekennzeichnet,**
**dass** die Schneidklinge (18; 28; 38; 48) innerhalb des Führungsschlitzes (17; 27; 37; 47) angeordnet und starr mit einer drehbaren Welle (15; 25; 35; 45) verbunden ist, welche rotierbar um eine senkrecht zur Ebene des Führungsschlitzes (17; 27; 37; 47) verlaufende Rotationsachse gelagert ist und durch eine Ausnehmung des Deckels (12; 22; 32; 42) senkrecht zur Ebene des Führungsschlitzes (17; 27; 37; 47) herausragt.

2. Schneidevorrichtung nach Anspruch 1, **dadurch gekennzeichnet, dass** eine Niederhalteeinrichtung (16) vorhanden ist, mittels welcher im Betriebszustand der Schneidevorrichtung Gewebe- oder Knorpelmaterial zur Herstellung dünner Scheiben auf die Grundfläche der ersten Vertiefung (13a; 23a; 33a; 43a) gedrückt werden kann.

3. Schneidevorrichtung nach Anspruch 2, **dadurch gekennzeichnet, dass** die Niederhalteeinrichtung (16) eine mechanische Höhenbegrenzung aufweist.

4. Schneidevorrichtung nach Anspruch 2 oder 3, **dadurch gekennzeichnet, dass** die Niederhalteeinrichtung (16) im Betriebszustand der Schneidevorrichtung manuell auf das Gewebe- oder Knorpelmaterial zur Herstellung dünner Scheiben gedrückt werden kann.

5. Schneidevorrichtung nach Anspruch 2 oder 3, **dadurch gekennzeichnet, dass** die Niederhalteeinrichtung (16) im Betriebszustand der Schneidevorrichtung mittels Federkraft automatisch auf das Gewebe- oder Knorpelmaterial zur Herstellung dünner Scheiben gedrückt wird.

6. Schneidevorrichtung nach einem der Ansprüche 1 bis 5, **dadurch gekennzeichnet, dass** die Rotationsachse der drehbaren Welle (25) ortsfest in der Schneidevorrichtung angeordnet ist.

7. Schneidevorrichtung nach einem der Ansprüche 1 bis 5, **dadurch gekennzeichnet, dass** die Rotationsachse der drehbaren Welle (15; 35; 45) in der Ebene des Führungsschlitzes (17; 37; 47) verschiebbar angeordnet ist.

8. Schneidevorrichtung nach Anspruch 7, **dadurch gekennzeichnet, dass** die Rotationsachse der drehbaren Welle (15; 35; 45) linear in der Ebene des Führungsschlitzes (17; 37; 47) verschiebbar oder auf einer gekrümmten Bahn, vorzugsweise auf einem Kreisbahnabschnitt, in der Ebene des Führungsschlitzes (17; 37; 47) verschiebbar angeordnet ist.

9. Schneidevorrichtung nach einem der Ansprüche 7 oder 8, **dadurch gekennzeichnet, dass** die drehbare Welle (15; 35) ein zumindest teilweise in den Führungsschlitz (17; 37) ragendes Ritzel aufweist, welches im Betriebszustand der Schneidevorrichtung in eine im Verbindungsabschnitt (19') angeordnete Zahnstange (19; 39) eingreift.

10. Schneidevorrichtung nach einem der vorhergehenden Ansprüche, **dadurch gekennzeichnet, dass** an dem der Schneidklinge (18; 28; 38) abgewandten Ende der drehbaren Welle (15; 25; 35) außerhalb des Deckels (12; 22; 32) ein Drehknopf (15'; 25'; 35') starr mit der drehbaren Welle (15; 25; 35) verbunden ist.

11. Schneidevorrichtung nach einem der vorhergehenden Ansprüche, **dadurch gekennzeichnet, dass** an dem der Schneidklinge (48) abgewandten Ende der drehbaren Welle (45) außerhalb des Deckels (42) ein Eingriffsteil (45'; 45") zum motorischen Antrieb der drehbaren Welle (45) starr mit dieser verbunden ist.

12. Schneidevorrichtung nach einem der vorhergehenden Ansprüche, **dadurch gekennzeichnet, dass** die rotierbare Schneidklinge (18; 28; 38; 48) eine ovale oder eine sichelartige oder eine balkenartige oder eine kreisrunde Form aufweist, insbesondere auf einem Kreisabschnitt, vorzugsweise auf einem Viertel- oder Halbkreis.

13. Schneidevorrichtung nach einem der vorhergehenden Ansprüche, **dadurch gekennzeichnet, dass** die Halteeinrichtung mehrere, im Betriebszustand der Schneidevorrichtung unterhalb des Führungsschlitzes (17; 37; 47) angeordnete Vertiefungen (13a; 33a; 43a; 13b; 33b; 43b; 33c; 43c) aufweist.

14. Schneidevorrichtung nach Anspruch 14, **dadurch gekennzeichnet, dass** die Vertiefungen geometrisch unterschiedlich gestaltet sind, insbesondere hinsichtlich ihrer jeweiligen Tiefe und/oder hinsichtlich ihrer jeweiligen Bodenfläche bezüglich Form und/oder Flächengröße und/oder konkaver, ebener oder konvexer Ausgestaltung und/oder hinsichtlich ihrem jeweiligen Begrenzungssteg bezüglich Form und/oder Größe.

15. Schneidevorrichtung nach einem der vorhergehenden Ansprüche, **dadurch gekennzeichnet, dass** an dem Verbindungsabschnitt (19') gegenüber dem ersten Arbeitsabschnitt (11a') mindestens ein zweiter Arbeitsabschnitt (11b'), insbesondere auch ein dritter Arbeitsabschnitt, jeweils mit dem gleichen gemeinsamen Führungsschlitz (17) anhängt, wobei jeder Arbeitsabschnitt (11a'; 11b') mindestens eine Vertiefung (13a; 13b) aufweist, und wobei die Vertiefungen (13a; 13b) auf einer Linie oder auf einer gekrümmten Kurve in einer parallel zur Ebene des Führungsschlitzes (17) verlaufenden Ebene angeordnet sind.

## Claims

1. Medical cutting apparatus (10; 20; 30; 40) for producing thin tissue or cartilage discs with an apparatus body (11; 21; 31; 41) and a cover (12; 22; 32; 42), wherein a first holding device is provided having a first working section (11a') with a first depression (13a; 23a; 33a; 43a) arranged on the upper side of the apparatus body (11; 21; 31; 41) and surrounded completely or partially by a first delimiting web (14), wherein the apparatus body (11; 21; 31; 41) has a connection section (19') to which the first working section (11a') is directly fixedly attached, wherein the cover (12; 22; 32; 42) has a counterpart (19") to the connection section (19') to which a first pressure section (11a") is directly fixedly attached, wherein the first pressure section (11a") in the operational state of the cutting apparatus is positioned opposed to the first depression (13a; 23a; 33a; 43a) of the first working section (11a') at an essentially uniform first distance defined by the geometrical shaping of the connection section (19') as well as its counterpart (19"), such that a guiding slot (17; 27; 37; 47) extending between the first working section (11a'; 21') and the first pressure section (11a") remains free for a cutting blade (18; 28; 38; 48),
**characterised in that**
the cutting blade (18; 28; 38; 48) is located within the guiding slot (17; 27; 37; 47) and fixedly connected to a rotatable shaft (15; 25; 35; 45), which is mounted rotatably about a rotation axis extending perpendicularly to the plane of the guiding slot (17; 27; 37; 47) and protrudes through a recess of the cover (12; 22; 32; 42) perpendicularly to the plane of the guiding slot (17; 27; 37; 47).

2. Cutting apparatus according to claim 1, **characterized in that** a hold-down device (16) is present by means of which, in the operational state of the cutting apparatus, tissue or cartilage material can be pressed onto the base surface of the first depression (13a; 23a; 33a; 43a) in order to produce thin discs.

3. Cutting apparatus according to claim 2, **characterized in that** the hold-down device (16) has a mechanical height limitation.

4. Cutting apparatus according to claim 2 or 3, **characterized in that** the hold-down device (16), in the operational state of the cutting apparatus, can be pressed manually onto the tissue or cartilage material to produce thin discs.

5. Cutting apparatus according to claim 2 or 3, **characterized in that** the hold-down device (16), in the operational state of the cutting apparatus, is pressed onto the tissue or cartilage material by spring force to produce thin discs.

6. Cutting apparatus according to any one of claims 1 to 5, **characterized in that** the rotation axis of the rotatable shaft (25) is located in the cutting apparatus in a stationary manner.

7. Cutting apparatus according to any one of claims 1 to 5, **characterized in that** the
rotation axis of the rotatable shaft (15; 35; 45) is displaceably located in the plane of the guiding slot (17; 37; 47) .

8. Cutting apparatus according to claim 7, **characterized in that** the rotation axis of the rotatable shaft (15; 35; 45) is located displaceably in the plane of the guiding slot (17; 37; 47) so as to be linearly displaceable in the plane of the guiding slot (17; 37; 47) or on a curved path, preferably on a circular path section.

9. Cutting apparatus according to any one of claims 7 or 8, **characterized in that** the rotatable shaft (15; 35) has a pinion protruding at least partially into the guiding slot (17; 37) which pinion, in the operational state of the cutting apparatus, engages with a tooth bar (19; 39) located in the connection section (19').

10. Cutting apparatus according to one of the preceding claims, **characterized in that** a rotary knob (15'; 25'; 35') is fixedly connected to the rotatable shaft (15; 25; 35) outside the cover (12; 22; 32) on the end of the rotatable shaft (15; 25; 35) facing away from the cutting blade (18; 28; 38).

11. Cutting apparatus according to one of the preceding claims, **characterized in that** an engagement part (45'; 45") for the motorized drive of the rotatable shaft (45) is fixedly connected to the rotatable shaft outside the cover (42) on the end of the rotatable shaft (45) facing away from the cutting blade (48).

12. Cutting apparatus according to one of the preceding claims, **characterized in that** the rotatable cutting blade (18; 28; 38; 48) has an oval or a crescent-like or a bar-like or a circular shape, in particular on a circular section, preferably on a quarter or half circle.

13. Cutting apparatus according to one of the preceding claims, **characterized in that** the holding device has a plurality of depressions (13a 33a; 43a; 13b; 33b; 43b; 33c; 43c) located below the guiding slot (17; 37; 47), in the operating state of the cutting apparatus.

14. Cutting apparatus according to Claim 14, **characterized in that** the depressions are of geometrically different design, in particular with regard to their respective depth and/or with respect to their respective base surface with respect to shape and/or surface size and/or concave, planar or convex configuration and/or with regard to their respective limitation web in terms of shape and/or size.

15. Cutting apparatus according to one of the preceding claims, **characterized in that** at least one second working section (11b'), in particular also a third working section, is attached to the connecting section (19') opposed to the first working section (11a'), in each case with the same common guiding slot (17), wherein each working section (11a'; 11b') has at least one depression (13a; 13b), and wherein the depressions (13a; 13b) are located on a line or on a curved bend in a plan extending in parallel to the plan of the guiding slot (17).

## Revendications

1. Dispositif de coupe médical (10 ; 20 ; 30 ; 40) pour la préparation tranches de tissu ou de cartilage minces comprenant un corps de dispositif (11 ; 21 ; 31 ; 41) et un couvercle (12 ; 22 ; 32 ; 42), dans lequel un premier dispositif de maintien est prévu, qui présente une première section de travail (11a') avec une première cavité (13a ; 23a ; 33a ; 43a) agencée sur le côté supérieur du corps de dispositif (11 ; 21 ; 31 ; 41), qui est complètement ou partiellement entouré par une première arête de limitation (14), dans lequel le corps de dispositif (11 ; 21 ; 31 ; 41) présente une section de connexion (19') à laquelle la première section de travail (11a') est fixée directement de manière rigide, dans lequel le couvercle (12 ; 22 ; 32 ; 42) présente une pièce complémentaire (19") à la section de connexion (19') à laquelle une première section de pression (11a") est fixée directement de manière rigide, dans lequel la première section de pression (11a"), dans l'état de fonctionnement du dispositif de coupe, est opposée à la première cavité (13a ; 23a ; 33a ; 43a) de la première section de travail (11a') avec une première distance sensiblement uniforme définie par la conception géométrique de la section de connexion (19') et de sa pièce complémentaire (19"), de sorte qu'une fente de guidage (17 ; 27 ; 37 ; 47) entre la première section de travail (11a' ; 21') et la première section de pression (11a") reste libre pour une lame de coupe (18 ; 28 ; 38 ; 48),
**caractérisé en ce que**
la lame de coupe (18 ; 28 ; 38 ; 48) est agencée à l'intérieur de la fente de guidage (17 ; 27 ; 37 ; 47) et reliée de manière rigide à un arbre rotatif (15 ; 25 ; 35 ; 45), qui est monté pouvant tourner autour d'un axe de rotation s'étendant perpendiculairement à un plan de la fente de guidage (17 ; 27 ; 37 ; 47), et fait saillie à travers un évidement du couvercle (12 ; 22 ; 32 ; 42) perpendiculairement au plan de la fente de guidage (17 ; 27 ; 37 ; 47).

2. Dispositif de coupe selon la revendication 1, **caractérisé en ce qu'**un dispositif de maintien (16) est prévu, au moyen duquel, dans l'état de fonctionnement du dispositif de coupe, de la matière tissulaire ou cartilagineuse pour préparer des tranches minces peut être pressée sur la surface de base de la première cavité (13a, 23a, 33a, 43a).

3. Dispositif de coupe selon la revendication 2, **caractérisé en ce que** le dispositif de maintien (16) présente une limitation de hauteur mécanique.

4. Dispositif de coupe selon la revendication 2 ou 3, **caractérisé en ce que** le dispositif de maintien (16), dans l'état de fonctionnement du dispositif de coupe, peut être pressé manuellement sur la matière tissulaire ou cartilagineuse pour la préparation de tranches minces.

5. Dispositif de coupe selon la revendication 2 ou 3, **caractérisé en ce que** le dispositif de maintien (16), dans l'état de fonctionnement du dispositif de coupe, est pressé au moyen d'une force élastique automatiquement sur la matière tissulaire ou cartilagineuse pour la préparation de tranches minces.

6. Dispositif de coupe selon l'une des revendications 1 à 5, **caractérisé en ce que** l'axe de rotation de l'arbre rotatif (25) est agencé de manière fixe dans le dispositif de coupe.

7. Dispositif de coupe selon l'une des revendications 1 à 5, **caractérisé en ce que** l'axe de rotation de l'arbre rotatif (15 ; 35 ; 45) est agencé de manière déplaçable dans le plan de la fente de guidage (17 ; 37 ; 47).

8. Dispositif de coupe selon la revendication 7, **caractérisé en ce que** l'axe de rotation de l'arbre rotatif (15 ; 35 ; 45) est agencé pouvant être déplacé linéairement dans le plan de la fente de guidage (17 ; 37 ; 47) ou est agencé pouvant être déplacé sur une trajectoire incurvée, de préférence sur une section de trajectoire circulaire, dans le plan de la fente de guidage (17 ; 37 ; 47).

9. Dispositif de coupe selon l'une des revendications 7 ou 8, **caractérisé en ce que** l'arbre rotatif (15 ; 35) présente un pignon faisant saillie au moins partiellement dans la fente de guidage (17 ; 37) qui, dans l'état de fonctionnement du dispositif de coupe, engrène dans une crémaillère (19 ; 39) agencée dans la section de connexion (19').

10. Dispositif de coupe selon l'une des revendications précédentes, **caractérisé en ce qu'**au niveau de l'extrémité de l'arbre rotatif (15 ; 25 ; 35) opposée à la lame de coupe (18 ; 28 ; 38) à l'extérieur du couvercle (12 ; 22 ; 32), un bouton rotatif (15'; 25', 35') est relié de manière rigide à l'arbre rotatif (15 ; 25 ; 35).

11. Dispositif de coupe selon l'une des revendications précédentes, **caractérisé en ce qu'**au niveau de l'extrémité de l'arbre rotatif (45) opposée à la lame de coupe (48) à l'extérieur du couvercle (42), une partie de mise en prise (45' ; 45") est reliée de manière rigide à celui-ci pour un entraînement par moteur de l'arbre rotatif (45).

12. Dispositif de coupe selon l'une des revendications précédentes, **caractérisé en ce que** la lame de coupe rotative (18 ; 28 ; 38 ; 48) présente une forme ovale ou de faucille ou de barre ou circulaire, en particulier sur une section circulaire, de préférence sur un quart ou un demi-cercle.

13. Dispositif de coupe selon l'une des revendications précédentes, **caractérisé en ce que** le dispositif de maintien, dans l'état de fonctionnement du dispositif de coupe, présente plusieurs cavités (13a ; 33a ; 43a ; 13b ; 33b ; 43b ; 33c ; 43c) disposées sous la fente de guidage (17 ; 37 ; 47).

14. Dispositif de coupe selon la revendication 14, **caractérisé en ce que** les cavités sont conçues géométriquement différentes, notamment en ce qui concerne leur profondeur respective et/ou en ce qui concerne leur surface inférieure respective en termes de forme et/ou de taille de surface et/ou de configuration concave, plane ou convexe et/ou en ce qui concerne leur rainure de délimitation respective en termes de forme et/ou de taille.

15. Dispositif de coupe selon l'une des revendications précédentes, **caractérisé en ce qu'**au niveau de la section de connexion (19') opposée à la première section de travail (11a') est fixée au moins une deuxième section de travail (11b'), en particulier également une troisième section de travail, chacune avec la même fente de guidage commune (17), dans lequel chaque section de travail (11a' ; 11b') présente au moins une cavité (13a ; 13b), et dans lequel les cavités (13a ; 13b) sont agencées sur une ligne ou sur une courbe incurvée dans un plan s'étendant parallèlement au plan de la fente de guidage (17).
